(19) Europäisches Patentamt
European Patent Office
Office européen des brevets

(11) **EP 2 833 168 A1**

(12) **EUROPEAN PATENT APPLICATION**

(43) Date of publication:
04.02.2015 Bulletin 2015/06

(51) Int Cl.:
**G02B 5/18** (2006.01)   **G02B 27/42** (2006.01)
**G01B 9/02** (2006.01)   **A61B 5/00** (2006.01)

(21) Application number: 14178723.4

(22) Date of filing: 28.07.2014

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: 30.07.2013 JP 2013157609

(71) Applicant: **CANON KABUSHIKI KAISHA
Ohta-ku
Tokyo 146-8501 (JP)**

(72) Inventor: **Kitamura, Tsuyoshi
Ohta-ku, Tokyo (JP)**

(74) Representative: **Houle, Timothy James
Canon Europe Ltd
European Patent Department
3 The Square
Stockley Park
Uxbridge, Middlesex UB11 1ET (GB)**

(54) **Transmission diffractive optical element and measuring device**

(57) A transmission diffractive optical element formed of silica glass for a wavelength band of 0.8 $\mu$m, includes a diffraction grating formed in the transmission diffractive optical element. If a refractive index of the silica glass is n, a wavelength of light entering a diffraction grating is $\lambda$ (nm), a pitch of the diffraction grating is p (nm), a depth of the diffraction grating is D (nm), and a duty ratio defined by a width of the diffraction grating divided by the pitch is $\alpha$, the pitch, the depth and the duty ratio of the diffraction grating satisfy predetermined conditions for acquiring high diffraction efficiency.

FIG. 1

**Description**

BACKGROUND OF THE INVENTION

Field of the Invention

The present invention relates to a transmission diffractive optical element and a measuring device.

Description of the Related Art

An optical coherence tomography (OCT) technique has been used for examining a fundus. A spectral-domain OCT (SD-OCT) is known as one of such systems for OCT. The SD-OCT detects the frequency distribution of light from a subject using a light source emitting a wide band wavelength light and a spectroscope, and performs Fourier transform of the detection signal to acquire a tomographic image in a depth direction. In the spectroscope a diffractive optical element separates interfering light between reference light and subject light from the fundus for each wavelength, and a sensor detects the light intensity (light quantity) for each wavelength. The OCT for examining the fundus uses a wavelength band of 0.8 $\mu$m as the wavelength of the light source, for example, a wide band center wavelength of 840 nm to 880 nm.

The diffraction efficiency of the diffractive optical element in the OCT affects the brightness of a sectional image to be acquired. A decrease in the diffraction efficiency at the edge of a wavelength band to be used leads to a deterioration in resolution in the sectional image. This is because the resolution is inversely proportional to the half width of a spectrum, so that a difference in the diffraction efficiency between a center wavelength and a wavelength at the edge of the wavelength band decreases the half width of the spectrum and increases (or deteriorates) the resolution. Therefore, it is important that the diffraction efficiency is higher not only in the center wavelength but also in the total area of the wavelength band in which the diffractive optical element performs spectroscopy. Since random polarized light enters the diffractive optical element, a high diffraction efficiency to both of transverse electric (TE) and transverse magnetic (TM) polarization is required of the diffractive optical element.

A transmission diffractive optical element emitting mainly low-order diffracted light generally has a higher diffraction efficiency among various types of diffractive optical elements. US patent No. 6747799 discusses conditions for a pitch, width, and height of a diffraction grating for realizing a diffraction efficiency of 90% or higher for both the TE and the TM polarization of a conventional (C) band (1530 nm to 1565 nm), for optical communication with respect to high diffraction efficiency of a transmission diffractive optical element. Japanese Patent No. 4749789 discusses a technique in which, in a diffraction grating, a thin film layer is provided immediately under the diffraction grating and a refractive index distribution of the thin film layer is brought into a distribution suited for the diffraction grating realizing a high diffraction efficiency. The wavelength of light entering the diffractive optical element according to Japanese Patent No. 4749789 is 1350 nm to 1750 nm.

US patent No. 6747799 discusses a condition for a grating for realizing a high diffraction efficiency for both the TE and the TM polarization in the C-band for optical communication. However, the condition does not realize a high diffraction efficiency for both the TE and the TM polarization at a wavelength band of 0.8 $\mu$m.

The wavelength of light entering the diffractive optical element according to Japanese Patent No. 4749789 is 1350 nm to 1750 nm. The diffractive optical element according to Japanese Patent No. 4749789 does not realize a high diffraction efficiency for both the TE and the TM polarization at a wavelength band of 0.8 $\mu$m either. Furthermore, a manufacturing process for forming the thin film layer according to Japanese Patent No. 4749789 is complicated and the production cost therefor increases. It is simpler to form the diffractive optical element by a single material without forming a thin film layer.

As stated above, a condition for the diffraction grating of the transmission diffractive optical element realizing a high diffraction efficiency for both the TE and the TM polarization at a wavelength band of 0.8 $\mu$m has not been known.

SUMMARY OF THE INVENTION

The present invention in its first aspect provides a transmission diffractive optical element as specified in claims 1 to 6.

Further features of the present invention will become apparent from the following description of embodiments with reference to the attached drawings. Each of the embodiments of the present invention described below can be implemented solely or as a combination of a plurality of the embodiments or features thereof where necessary or where the combination of elements or features from individual embodiments in a single embodiment is beneficial.

BRIEF DESCRIPTION OF THE DRAWINGS

Fig. 1 illustrates parameters of a diffractive optical element.
Fig. 2 is a schematic diagram illustrating the diffraction of a diffractive optical element.
Figs. 3A, 3B, and 3C illustrate calculation results of diffraction efficiency at the time when the number of grooves of a diffraction grating is 1200.
Figs. 4A, 4B, and 4C illustrate calculation results of diffraction efficiency at the time when the number of grooves of a diffraction grating is 1400.
Figs. 5A, 5B, 5C, and 5D illustrate calculation results of diffraction efficiency at each number of grooves.
Fig. 6 is a chart illustrating a grating depth and a duty ratio which define straight lines A and B.
Fig. 7 is a chart illustrating curves C and D.
Fig. 8 illustrates results of calculation of diffraction efficiency at a predetermined wavelength band of a diffractive optical element according to a first embodiment.
Fig. 9 is a diagram illustrating the slanted side faces of the elongate portions of a diffraction grating according to a second embodiment.
Fig. 10 illustrates results of calculation of diffraction efficiency at a predetermined wavelength band according to the second embodiment.
Fig. 11 is a schematic diagram illustrating an optical coherence tomography measuring device according to a third embodiment.

DESCRIPTION OF THE EMBODIMENTS

Various embodiments, features, and aspects of the invention will be described in detail below with reference to the drawings.

A first embodiment describes a diffractive optical element used for optical coherence tomography (OCT) for examining a fundus.

The OCT technique used for examining a fundus can acquire a sectional image and a stereoscopic image in various places on an eye ball such as a retina, anterior eye cornea, angle, and iris, and has an excellent effect particularly in diagnosis of retinal disease such as macular degeneration and glaucoma. The OCT technique widely used in recent years is referred to as a spectral domain (SD) OCT. The SD-OCT detects the frequency distribution of light from a subject using a light source emitting a wide band wavelength light and a spectroscope, and performs Fourier transform of the detection signal to acquire a tomographic image in a depth direction. In the spectroscope a diffractive optical element splits (disperses) interfering light between reference light and subject light from the fundus for each wavelength, and a charge-coupled device (CCD) sensor detects the light intensity (light quantity) for each wavelength. The diffractive optical element realizes spectroscopy such that light with a specific wavelength, among light with a certain wavelength bandwidth that has entered a diffraction grating, is transmitted or reflected in a specific direction using a diffraction phenomenon of light. The OCT technique used for examining a fundus uses a wavelength band of 0.8 $\mu$m as the wavelength of a light source, for example, a wide band of the center wavelength of 840 nm to 880 nm. For this reason, a diffractive optical element adapted to the wavelength band of 0.8 $\mu$m is used in the spectroscope. The system gains an advantage in speed of measurement and image processing, spatial resolution, and detection sensitivity, in comparison with a time domain (TD) OCT technique that acquires a sectional image in the depth direction of a detection object while mechanically moving a reference mirror of an interferometer.

One of the features required for a diffractive optical element in consideration of the performance of an image acquired by an OCT is diffraction efficiency. The diffraction efficiency is a value indicating the ratio between the quantity of light diffracted to a desired order and the quantity of incident light. Because the diffraction efficiency contributes to the brightness of an image, it is desirable that the diffraction efficiency is high.

The diffraction efficiency includes a polarization dependability of incident light. Light from a light source emitting light with a wide-band wavelength used in a SD-OCT technique generally has no specific vibration direction with respect to a polarization. Therefore, it is desirable that the diffractive optical element has a high diffraction efficiency for both the TE and the TM polarization (s polarization and p polarization).

The diffraction efficiency differs according to the wavelength of incident light. For example, even if the diffraction efficiency is high at a specific center wavelength and if the diffraction efficiency is low at a wavelength at the end of a wide wavelength band, the resolution of an image acquired after performing Fourier transform on a detection signal detected by the CCD sensor is deteriorated to lower measurement accuracy. For this reason, the diffraction efficiency needs to be high not only for a specific wavelength but also for at least all the wavelength band to be used.

In general, a transmission diffractive optical element is used as the diffractive optical element for SD-OCT. This is because the transmission diffractive optical element generally has a higher diffraction efficiency than a reflection diffractive optical element. A diffractive optical element formed by directly etching onto a silica glass substrate with high purity is known as one configuration of the transmission diffractive optical element. Little light is lost on a surface opposite to the surface where the diffraction grating is formed because a reflection prevention film is coated on the surface opposite to the surface where the diffraction grating is formed. For this reason, almost all losses in light in the transmission diffractive optical element are equal to light propagated by the diffraction grating at other orders excluding a desired order. On the other hand, a metal film coating with a reflection rate of approximately 90% is used as a reflection layer in the reflection type diffractive optical element, and light losses in the reflection layer are great, so that the reflection diffractive optical element is lower in diffraction efficiency than the transmission diffractive optical element.

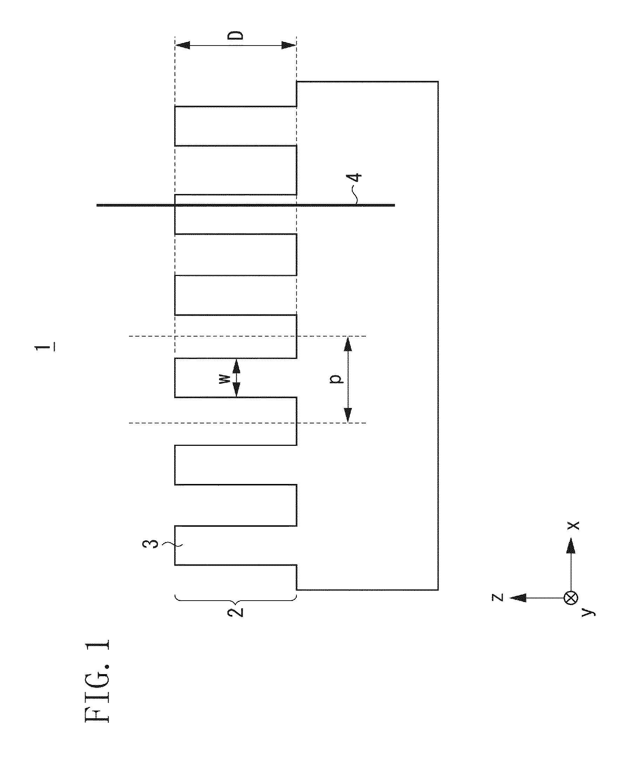

Fig. 1 is a cross section of a transmission diffractive optical element 1 according to the present embodiment. The transmission diffractive optical element 1 is formed such that a resist coated on a silica glass (or quartz) substrate is exposed by an exposure device, and the exposed substrate is developed and etched. This method can form the transmission diffractive optical element by a simpler process than forming a thin film layer according to Japanese Patent No. 4749789. A diffraction grating 2 whose cross section is uneven is formed in the transmission diffractive optical element 1. The diffraction grating 2 is formed of a plurality of elongate portions (elongate portion 3) and grooves between the elongate portions. In the present embodiment, each elongate portion 3 of the diffraction grating 2 is rectangular. The elongate portion 3 of the same sectional shape extends in the y direction, and a plurality of the elongate portions 3 are repetitively arranged in the x direction to form a line and space diffraction grating 2.

Parameters defining the shape of the diffractive optical element 1 illustrated in Fig. 1 will be described below. The elongate portions 3 of the diffraction grating 2 and the grooves between the elongate 3 portions are arranged in one direction at an equally spaced pitch (or distance) p to diffract incident light. In other words, the pitch p is equal to the sum of the width of an elongate portion and the width of an adjacent groove in the direction in which the elongate portions 3 of the diffraction grating 2 are repetitively arranged. A reciprocal of the pitch p of the diffraction grating is referred to as the number of grooves and usually indicated by the number of grooves per millimeter. The length of the diffraction grating 2 (the elongate portion 3) in the direction of a grating normal 4 (z direction) perpendicular to a diffraction grating surface on which the diffraction grating 2 is formed is defined as a grating depth D. The width of the elongate portion 3 in the direction in which the elongate portions 3 of the diffraction grating 2 are repetitively arranged (x direction) is defined as a grating width w. A value (w/p) in which the grating width w is divided by the pitch p is referred to as a duty ratio $\alpha$ (or duty cycle). The shape of the diffraction grating 2 is uniquely determined by defining the pitch p or the number of grooves, the grating depth D, and the duty ratio $\alpha$.

The pitch p or the number of grooves, the grating depth D, and the duty ratio $\alpha$ decide the order to which diffraction light is allocated, that is, significantly affect the diffraction efficiency. Therefore, by appropriately setting the pitch p or the number of grooves, the grating depth D, and the duty ratio $\alpha$, a high diffraction efficiency can be realized. In the present embodiment, the three parameters of the pitch p or the number of grooves, the grating depth D, and the duty ratio $\alpha$ were changed by simulation using a computer to calculate the diffraction efficiency. The calculation is performed using a rigorous coupled-wave analysis (RCWA) method which is a type of an electromagnetic field analysis method.

The wavelength of incident light needs to be defined for the calculation. Because the light source used in SD-OCT practically applied at present has a wavelength band of 0.8 $\mu$m whose center wavelength is 840 nm to 880 nm, the diffraction efficiency was calculated for the light of a specific wavelength 855 nm in this wavelength band in the present embodiment. The material for forming the diffractive optical element was a single material of silica glass. Silica glass is very high in transmissivity at a wavelength band of 0.8$\mu$m, excellent in chemical stability, and inexpensive. It has been well known that silica glass is used as a transmission diffractive optical element such that a diffraction grating structure is provided by performing etching on a surface of the silica glass.

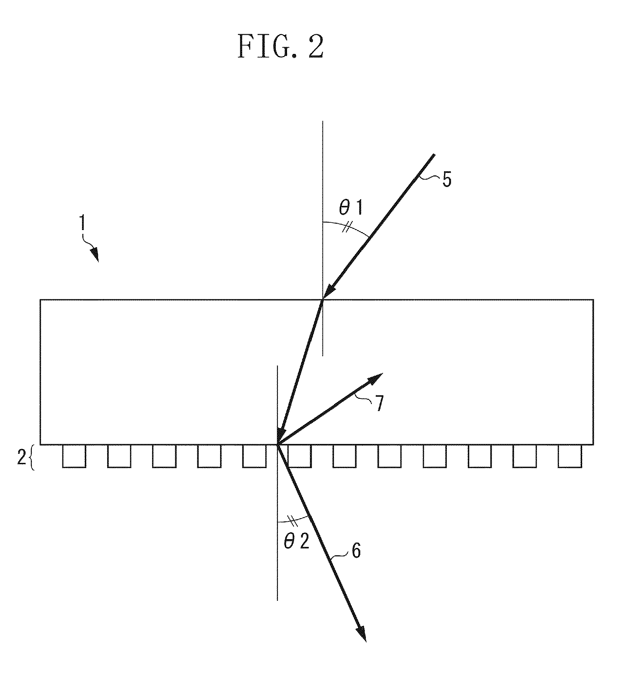

Incident and emitting angles of light were calculated in a Littrow arrangement (condition). As illustrated in Fig. 2, incident light 5 enters the surface opposite to the surface on which the diffraction grating 2 is formed, is transmitted by the diffractive optical element 1, is diffracted by the diffraction grating 2, and exits as diffracted light 6. The Littrow arrangement refers to an arrangement in which an incident angle $\theta1$ of the incident light 5 is equal to an emitting angle $\theta2$ of the diffracted light 6 of a desired order. Since the emitting angle of the diffracted light differs according to the wavelength thereof, a device is designed such that the Littrow arrangement holds true for a certain wavelength, for example, the center wavelength in a wavelength band to be used.

The relationship between the incident angle $\theta1$ of the incident light 5 and the emitting angle $\theta2$ of the diffracted light 6 can be expressed by an equation $m \times \lambda = p \times (\sin\theta1 + \sin\theta2)$, where a wavelength is $\lambda$(nm), a diffraction order is m, and a pitch of the diffraction grating 2 is p(nm). In the Littrow arrangement, an equation $m \times \lambda = 2p \times \sin\theta$ is established when $\theta1$ is equal to $\theta2$ and a Littrow angle $\theta$ is defined as $\theta = \theta1 = \theta2$.

In the present embodiment, a diffraction efficiency related to a primary diffracted light was calculated with the diffracted light 6 of the desired order as the primary diffracted light. This is because the use of a secondary or higher order of the diffracted light separates light into each order, and a high diffraction efficiency cannot be realized. In the present embodiment, the parameters of the shape of the diffraction grating 2 are set such that the light quantity of the primary diffracted light among diffracted lights is maximized, and the diffraction efficiency related to the primary diffracted light is maximized.

In a case where diffraction occurs in such a manner that the primary diffracted light exits into air from the diffractive optical element 1, a condition exists under which a secondary diffracted light 7 is reflected on the surface on which the diffraction grating 2 is formed. In a case where the secondary diffracted light 7 occurs (exists), an equation $2\lambda = p \times (\sin\theta + n \times \sin\theta3)$ is established, where the emitting angle (Littrow angle) of the primary diffracted light is $\theta$, the emitting angle of a secondary reflection diffracted light in a case where the secondary reflection diffracted light 7 occurs is $\theta3$, and the refractive index of the diffractive optical element 1 is n. On the other hand, $\theta$ is the emitting angle of the primary diffracted light 6, so that an equation $\lambda = 2p \times \sin\theta$ is established. The two equations produce $\sin\theta3 = 3\lambda/(2n \times p)$. If $0 \leqq \sin\theta3 \leqq 1$, a $\theta3$ value exists, so that the secondary diffracted light 7 exists if a condition of $0 \leqq 3\lambda/(2n \times p) \leqq 1$ is satisfied. In other words, if $3\lambda/(2n \times p) > 1$, the secondary diffracted light 7 does not exist. For this reason, it is required to set a pitch p to satisfy $2n \times p < 3\lambda$ so that a high diffraction efficiency is realized without causing the secondary diffracted light 7.

If a refractive index n of 1.45 and a wavelength $\lambda$ of 855 nm are substituted in the above equation $2n \times p < 3\lambda$, 2p (double of the pitch p) needs to be 844 nm or less, or 1131/mm or more in terms of the number of the grooves.

The above has described the diffraction of light exiting into air from the diffractive optical element 1. However, also in case where the diffraction of light falling on the diffractive optical element 1 is from air, the second transmission diffracted light occurs under the same conditions. Therefore, a condition of $2n \times p < 3\lambda$ needs to be similarly satisfied for a high diffraction efficiency.

In simulation, the number of grooves of 1150 to 1500 was used in consideration of the above condition related to the pitch or the number of the grooves s. The calculation was performed while the grating depth D was changed from 1 $\mu$m to 3$\mu$m and the duty ratio $\alpha$ was changed from 0.3 to 0.8. It is ineffective to increase the grating depth more than required or select a duty ratio largely deviating from 0.5, in terms of technique and cost in consideration of the production of the diffractive optical element, so that the grating depth D and the duty ratio $\alpha$ were set to the above range.

The diffraction efficiency acquired by calculation is a ratio of the light quantity of the primary transmission diffracted light to the total light quantity before transmission when transmission diffraction occurs in the diffraction grating 2 under the Littrow arrangement condition, and influence due to the surface opposite to the surface of the diffraction grating is not included. As described above, in general, there is no specific oscillation direction with regard to polarization in the light source for the SD-OCT, and a high diffraction efficiency for both polarizations is required for the diffractive optical element. For this reason, an average diffraction efficiency for both polarizations was calculated.

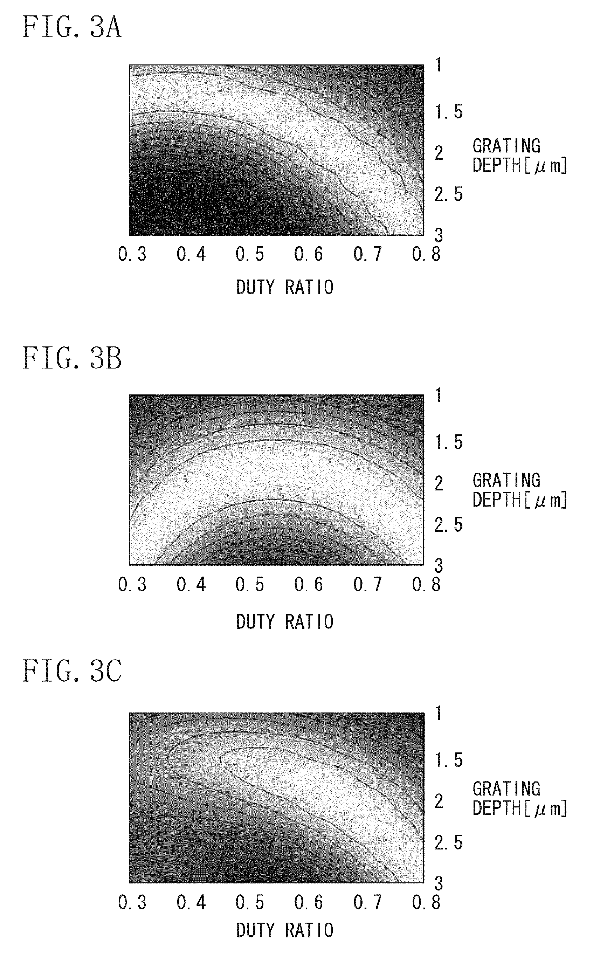

Figs. 3A to 3C, Figs. 4A to 4C, and Figs. 5A to 5D illustrate calculation results. Figs. 3A to 3C illustrate the diffraction efficiency for the grating depth D and the duty ratio $\alpha$ in a case where the number of grooves of the diffraction grating is 1200. Fig. 3A illustrates the diffraction efficiency for the TM polarization. Fig. 3B illustrates the diffraction efficiency for the TE polarization. Fig. 3C illustrates an average value of the diffraction efficiency for both polarizations. Figs. 3A to 3C indicate that the value increases as black changes into white and a contour line is illustrated. As is clear from Figs. 3A and 3B, the diffraction efficiency for the grating depth and the duty ratio differs according to polarization. In the case of the grating depth and the duty ratio acquired when both polarizations are high in diffraction efficiency, both polarizations are high in average diffraction efficiency.

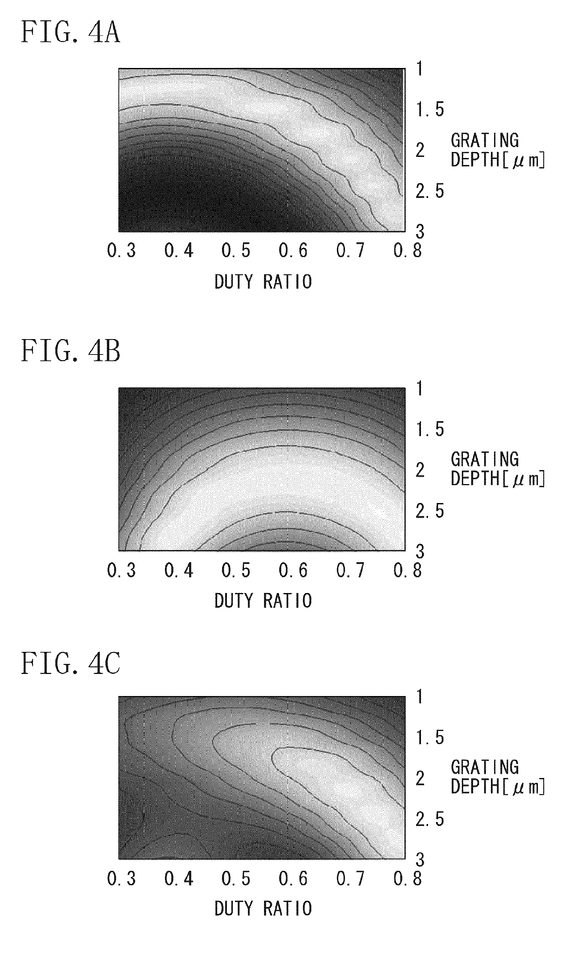

Fig. 4 is a chart illustrating the diffraction efficiency for the grating depth D and the duty ratio $\alpha$ in a case where the number of grooves of the diffraction grating is 1400. Fig. 4A illustrates the diffraction efficiency for the TM polarization. Fig. 4B illustrates the diffraction efficiency for the TE polarization. Fig. 4C illustrates an average value of the diffraction efficiency for both polarizations. When Fig. 3A is compared with Fig. 4A, the dependence of the diffraction efficiency on the grating depth D and the duty ratio $\alpha$ is substantially unchanged for the TM polarization. However, when Fig. 3B is compared with Fig. 4B, the dependence of the diffraction efficiency on the grating depth D and the duty ratio $\alpha$ is different for the TE polarization. Specifically, the grating depth when the diffraction efficiency is high in Fig. 4B tends to be greater than the grating depth when the diffraction efficiency is high in the diffraction efficiency in Fig. 3B. The same holds true of the duty ratio. The dependence of the average diffraction efficiency on the grating depth D and the duty ratio $\alpha$ also changes in a similar manner. When Fig. 3C is compared with Fig. 4C, as is the case with the TM polarization, also with regard to the average diffraction efficiency, it is clear that both of the grating depth D and the duty ratio $\alpha$ which are high in the diffraction efficiency increase as the number of grooves of the diffraction grating is increased.

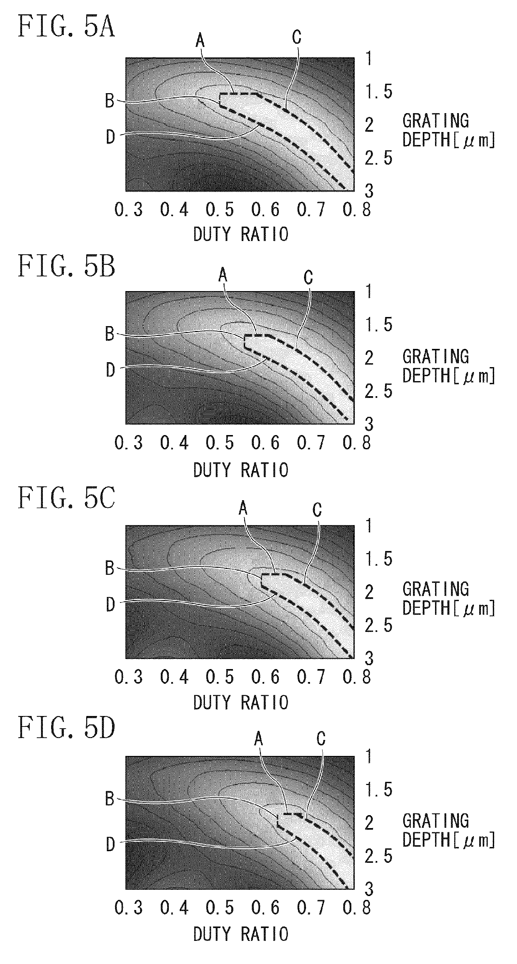

Figs. 5A to 5D illustrate the dependence of the average diffraction efficiency on the grating depth and the duty ratio for both polarizations. Fig. 5A illustrates the dependence of the average diffraction efficiency on the grating depth and the duty ratio for both polarizations in a case where the number of the grooves of the diffraction grating is 1200. Figs. 5B, 5C, and 5D illustrate the dependence thereof in a case where the number of grooves of the diffraction grating is 1300, 1400, and 1500 respectively.

In general, the maximum diffraction efficiency of a conventional diffractive optical element is approximately 90% in a wavelength band to be used. For this reason, in the present embodiment, a diffraction efficiency of 93% is high enough as a calculation efficiency for realizing a diffraction efficiency higher than a conventional efficiency. In the present embodiment, therefore, we focus attention on the condition that the average diffraction efficiency for both polarizations is 93% or more and define the values of parameters satisfying the condition.

As results of surveying an area where the average diffraction efficiency for both polarizations is 93% or more, it can be seen from Figs. 5A to 5D that the area can be defined by an area encompassed by two straight lines A and B and two secondary curves C and D in the calculation results for each of the numbers of grooves of the diffraction grating. The straight line A defines the lower limit of the grating depth D satisfying the above condition and is defined by the grating depth D with a certain value. The straight line B defines the lower limit of the duty ratio satisfying the above condition and is defined by the duty ration $\alpha$ with a certain value.

The straight lines A and B have dependency on the number of grooves of the diffraction grating. On the other hand, the secondary curves C and D have little dependency on the number of grooves of the diffraction grating. Because the number of grooves of the diffraction grating is a reciprocal of the pitch p, the straight line A is expressed as a function of the grating depth D and the pitch p and the straight line B is expressed as a function of the duty ratio $\alpha$ and the pitch p. The curves C and D are expressed as functions of the grating depth D and the duty ratio $\alpha$.

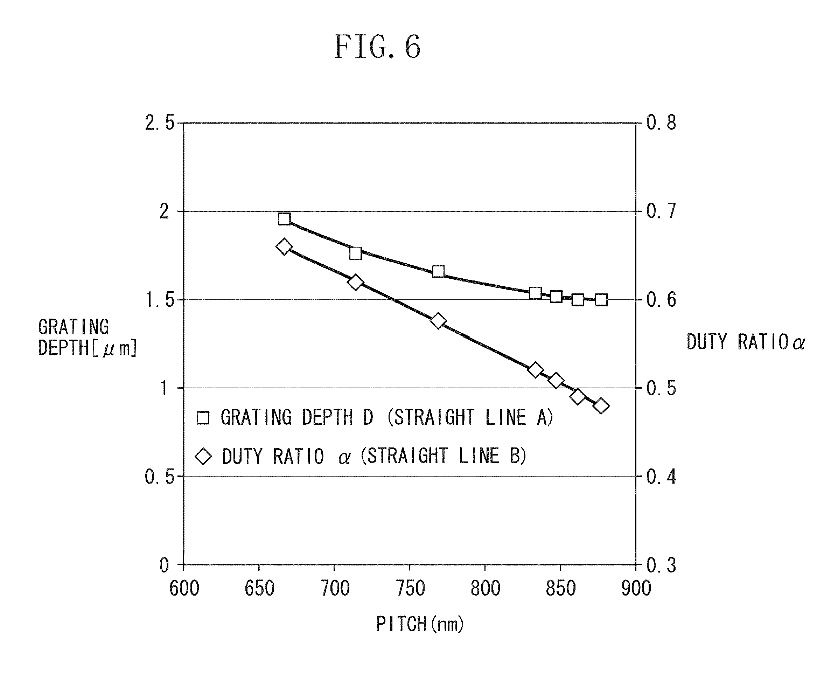

Fig. 6 is a chart in which the grating depth D ($\mu$m) defining the straight line A and the duty ratio $\alpha$ defining the straight line B are plotted relative to the number of grooves of the diffraction grating, i.e., a reciprocal of the pitch p (nm).

If the grating depth D defining the straight line A is approximated by a quadratic function, a relationship between the grating depth D ($\mu$m) and the pitch p (nm) defining the straight line A can be expressed by the following equation 1:

$$D = 7.8638 \times 10^{-6} \times p^2 - 1.4279 \times 10^{-2} \times p + 7.9734 \ \ldots(1).$$

If the duty ratio $\alpha$ defining the straight line B is approximated by a linear function, a relationship between the duty ratio $\alpha$ and the pitch p defining the straight line B can be expressed by the following equation 2:

$$\alpha = - 8.5747 \times 10^{-4} \times p + 1.2328 \ \ldots(2).$$

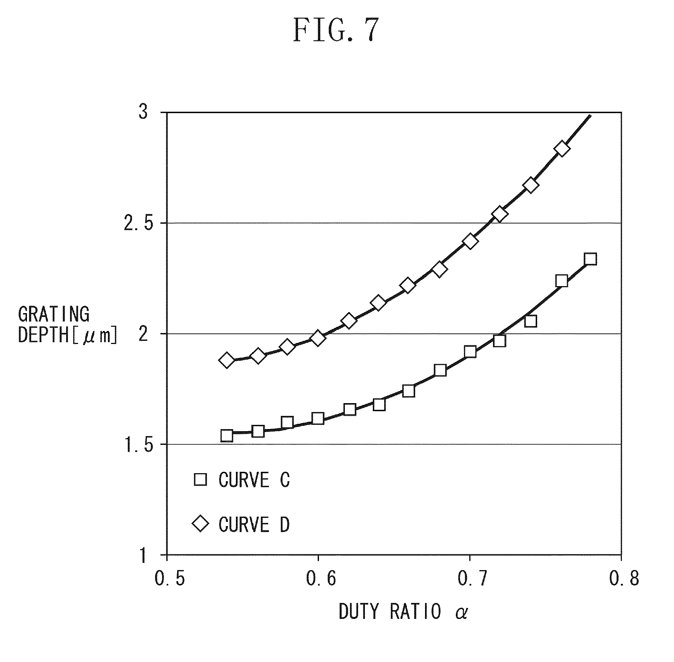

Fig. 7 is a chart illustrating a relationship between the grating depth D ($\mu$m) and the duty ratio $\alpha$ defining the curves C and D. If the relationship between the grating depth D ($\mu$m) and the duty ratio $\alpha$ is approximated by a quadratic function, a curve C can be expressed by the following equation 3:

$$C = 13.19 \times \alpha^2 - 14.16 \times \alpha + 5.360 \ \ldots(3).$$

A curve D can be expressed by the following equation 4:

$$D = 15.44 \times \alpha^2 - 15.73 \times \alpha + 5.870 \ \ldots(4).$$

As described above, the relation does not substantially depend on the number of grooves of the diffraction grating.

The parameters of the diffraction grating capable of attaining the average diffraction efficiency of 93%, or more, for both polarizations needs to satisfy the following five inequalities, where, inequalities 6 to 9 represent an area encompassed by the straight lines A and B and the curves C and D:

$$2n \times p < 3\lambda \ \ldots(5)$$

$$D > 7.8638 \times 10^{-6} \times p^2 - 1.4279 \times 10^{-2} \times p + 7.9734 \ \ldots(6)$$

$$\alpha > -8.5747 \times 10^{-4} \times p + 1.2328 \quad ...(7)$$

$$D > 13.19 \times \alpha^2 - 14.16 \times \alpha + 5.360 \quad ...(8),$$

and

$$D < 15.44 \times \alpha^2 - 15.73 \times \alpha + 5.870 \quad ...(9).$$

As described above, a high diffraction efficiency is required of the diffractive optical element for SD-OCT throughout the wavelength band to be used. The above calculation was made using a wavelength of 855 nm. However, the following describes that a high diffraction efficiency can be achieved even at a wavelength band including wavelengths other than 855 nm as far as the shape of the diffraction grating satisfies the above inequalities.

As an example, the number of grooves of the diffraction grating of 1200, a pitch of 833.3 nm, a grating depth of 2.06 $\mu$m, and a duty ratio of 0.66 are set as the parameters satisfying the above inequalities. The diffraction efficiency in this case was calculated. At the wavelength of 855 nm, the diffraction efficiency for the TE polarization is 97.6%, the diffraction efficiency for the TM polarization is 98.1%, and the average diffraction efficiency for both polarizations is 97.9%.

Although, wavelength bands used in SD-OCT are various in the vicinity of 800 nm, in the present embodiment, we assumed that light with wavelengths of 795 nm to 915 nm fell within the wavelength band, and the diffraction efficiency was calculated at the wavelength band. The incident angle of the light was 30.86 degrees, that is the Littrow arrangement condition at the center wavelength of 855 nm.

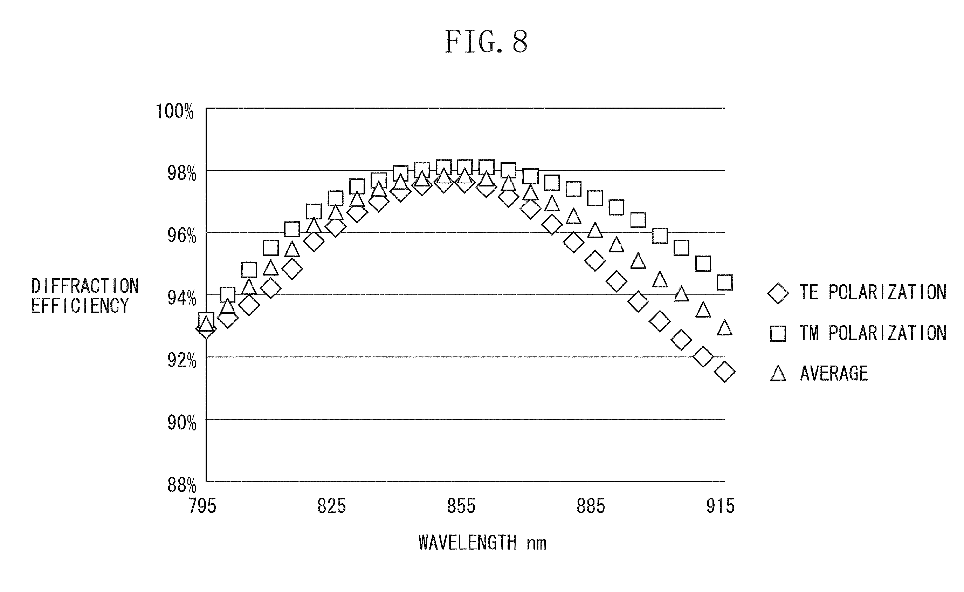

Fig. 8 illustrates the results of the calculation of the diffraction efficiency at the wavelength band of 795 nm to 915 nm under the above condition. The average diffraction efficiency for both the TE and TM polarizations at the edges of the wavelength band of 795 nm and 915 nm is 93.0%, which is high enough as compared with the diffraction efficiency at the same wavelength band, of a generally available conventional diffractive optical element. The parameters of the diffraction grating are thus set to change the diffraction efficiency as a quadratic function in which the efficiency value reaches a peak at a set wavelength 855 nm at the wavelength band. The parameters of the diffraction grating at which a high diffraction efficiency is obtained at the set wavelength of 855 nm are selected, and thereby, a high diffraction efficiency can be realized in the wavelength band to be used.

From the above, if the diffractive optical element has the diffraction grating defined by the three parameters of the pitch p or the number of grooves of the diffraction grating, the grating depth D, and the duty ratio $\alpha$ satisfying the above inequalities, the diffractive optical element can realize a high diffraction efficiency for both the TE and TM polarizations with a wavelength band of 0.8 $\mu$m.

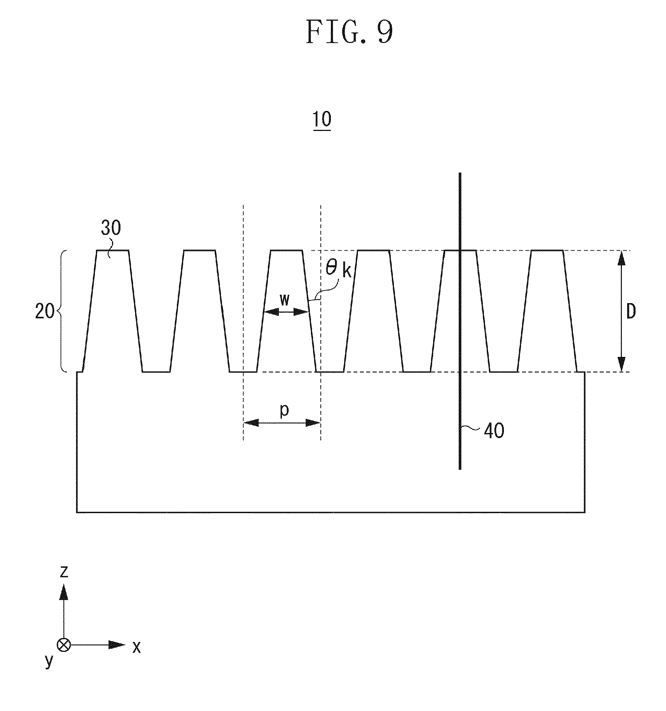

A diffractive optical element 10 according to a second embodiment will be described below. In the first embodiment, the cross section of the elongate portion of the diffraction grating is rectangular. In the present embodiment, the cross section of the elongate portion of the diffraction grating is not rectangular, and the side faces thereof are tilted. For example, if the diffraction grating is formed by etching, the side faces of the elongate portion are formed not vertically but at an angle. In this case, as illustrated in Fig. 9, a cross section of an elongate portion 30 of a diffraction grating 20 is of an isosceles trapezoid shape. The side faces of the elongate portion 30 are angled, or inclined, with respect to a grating normal 40 and have an angle $\theta k$ of several degrees. In other words, the width of the elongate portion 30 changes in the depth direction of the diffraction grating 20. The pitch p and the grating depth D are similar to those in the first embodiment. A grating width w, however, is not constant in the direction of the grating normal 40, so that the grating width w is defined as a width in a direction in which the elongate portion 30 is arranged (in the x direction) at a position half of the grating depth D. Thus, a duty ratio is determined using the width of the elongate portion 30 in a predetermined depth of the diffraction grating 20.

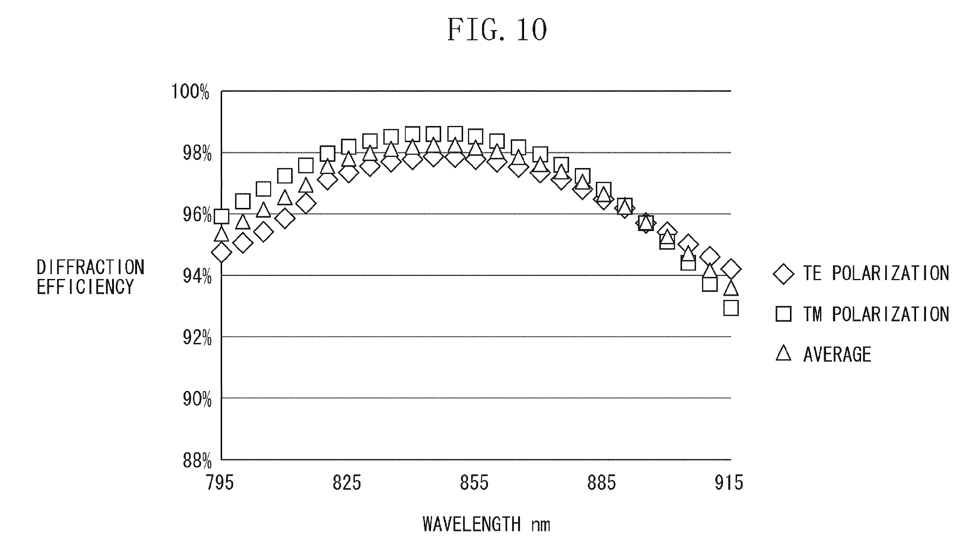

Fig. 10 illustrates results of calculating the dependence of the wavelength on the diffraction efficiency using the inclination angle $\theta k$ of 4 degrees. As is the case with the calculation according to the first embodiment, the calculation was performed under the conditions of the number of grooves of the diffraction grating of 1200, a pitch of 833.3 nm, a grating depth of 2.06 $\mu$m, a duty ratio of 0.66, and the incident angle of 30.86 degrees.

The average diffraction efficiency for both polarizations was 97.6% at a set wavelength of 855 nm, 94.7% at the one edge of the wavelength band of 795 nm, and 93.0% at the other edge of the wavelength band of 915 nm. Thereby, even if the side faces of the elongate portion of the diffraction grating have a tilt, or inclined, angle, the diffractive optical element can realize a high diffraction efficiency for both the TE and TM polarizations with a wavelength band of

0.8 $\mu$m as long as the diffractive optical element has the diffraction grating defined by the parameters satisfying the above inequalities.

A third embodiment describes a measuring device (an SD-OCT apparatus and an ophthalmologic apparatus) using the above transmission diffractive optical element and an optical coherence tomography method.

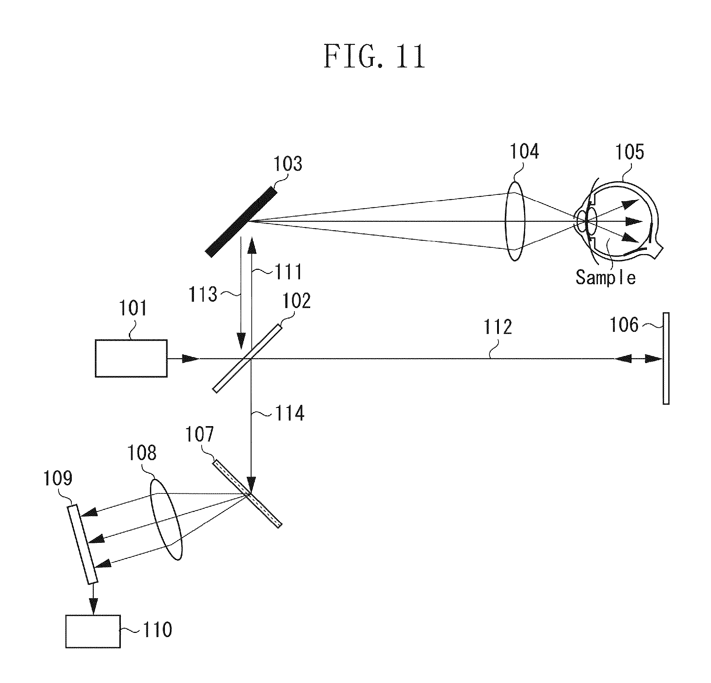

The configuration of the SD-OCT apparatus according to the present embodiment will be described below. Fig. 11 illustrates the configuration of the SD-OCT apparatus according to the present embodiment. Light having a wavelength band of 0.8 $\mu$m emitted from a light source 101 is split into a reference light 112 and a measurement light 111 by a beam splitter 102. The measurement light 111 with which an observation target (a subject: an eyeball 105) is irradiated through a lens 104 is returned as a return light 113 by reflection and scatter. The return light 113 and the reference light 112 are combined into an interfering light 114 by the beam splitter 102. The interfering light 114 is dispersed by a diffractive optical element 107 and passes through a lens 108, and irradiates a sensor 109. The sensor 109 converts the intensity of the interfering light into an analog electric signal for each wavelength (photoelectric conversion), and outputs the analog electric signal to an image information processing unit 110. The image information processing unit 110 converts the input analog electric signal into a digital electric signal (A/D conversion), and acquires a tomographic image of a subject by calculation processing based on the digital electric signal for each wavelength. Specifically, the image information processing unit 110 applies processing such as Fourier transform processing to the digital electric signal for each wavelength to acquire a tomographic image of the eyeball 105. This enables examination of the fundus of the eyeball 105.

The transmission diffractive optical element according to the first or second embodiments is used as the diffractive optical element 107. The diffractive optical element 107 is used in the Littrow arrangement. The diffractive optical element according to the first or second embodiments can realize a high diffraction efficiency for both the TE and TM polarizations in a wavelength band of 0.8 $\mu$m, so that the resolution of an image acquired by the SD-OCT apparatus is decreased to allow high accuracy measurement.

While the present invention has been described with reference to embodiments, it is to be understood that the invention is not limited to the disclosed embodiments. The scope of the following claims is to be accorded the broadest interpretation so as to encompass all such modifications and equivalent structures and functions.

**Claims**

1. A transmission diffractive optical element (1) formed of silica glass for a wavelength band of 0.8 $\mu$m, comprising:

a diffraction grating (2) formed in the transmission diffractive optical element (1),
wherein if a refractive index of the silica glass is n, a wavelength of light entering the diffraction grating (2) is $\lambda$ (nm), a pitch of the diffraction grating (2) is p (nm), a depth of the diffraction grating (2) is D (nm), and a duty ratio defined by a width w of the diffraction grating (2) divided by the pitch is $\alpha$, the pitch, the depth and the duty ratio of the diffraction grating (2) satisfy the following equations:

$$2n \times p < 3\lambda,$$

$$D > 7.8638 \times 10^{-6} \times p^2 - 1.4279 \times 10^{-2} \times p + 7.9734 \quad \alpha > -8.5747 \times 10^{-4} \times p + 1.2328,$$

$$D > 13.19 \times \alpha^2 - 14.16 \times \alpha + 5.360,$$

and

$$D < 15.44 \times \alpha^2 - 15.73 \times \alpha + 5.870.$$

2. The transmission diffractive optical element (1) according to claim 1, wherein

a light quantity of a primary diffracted light among light diffracted by the diffraction grating (2) is a maximum, and an emitting angle of the primary diffracted light at a particular wavelength is equal to an incident angle of light entering the diffraction grating.

3.  The transmission diffractive optical element (1) according to claim 1 or 2, wherein a center wavelength of light entering the diffraction grating (2) is 840 nm to 880 nm.

4.  The transmission diffractive optical element (1) according to any one of claims 1 to 3, wherein the diffraction grating includes elongate portions (3) and grooves alternately arranged, a cross section of each elongate portion (3) is of a trapezoid shape, and the duty ratio, in which the width at a position half of the depth of the diffraction grating (2) is divided by the pitch, satisfies the equations.

5.  The transmission diffractive optical element (1) according to any one of claims 1 to 3, wherein the diffraction grating includes elongate portions (3) and grooves alternately arranged, the width of the elongate portions (3)) changes in a depth direction of the diffraction grating (2), and the duty ratio, in which the width of the diffraction grating (2) at a predetermined depth of the diffraction grating (2) is divided by the pitch, satisfies the equations.

6.  The transmission diffractive optical element (1) according to any one of claims 1 to 3, wherein the diffraction grating includes elongate portions (3) and grooves alternately arranged, and the elongate portions (3) are rectangular in a cross section.

7.  The transmission diffractive optical element (1) according to claim 1, wherein the diffraction grating includes elongate portions (3) and grooves alternately arranged, and wherein
the depth of the diffraction grating is the length of the diffraction grating (2) in a grating normal (4) direction,
the width of the diffraction grating is the width of an elongate portion (3) in the direction in which the elongate portions 3 of the diffraction grating 2 are repetitively arranged, and
the pitch of the diffraction grating is the sum of the width of an elongate portion and the width of an adjacent groove in the direction in which the elongate portions 3 of the diffraction grating 2 are repetitively arranged.

8.  A measuring device for measuring a subject such that the subject is irradiated with light having a wavelength band of 0.8 $\mu$m, the measuring device comprising:

    the transmission diffractive optical element (1) according to any one of claims 1 to 7 configured to disperse light from the subject; and
    a sensor (9) configured to detect the light dispersed by the transmission diffractive optical element (1).

9.  The measuring device according to claim 8, wherein the transmission diffractive optical element (1) is arranged in a Littrow arrangement.

10. The measuring device according to claim 8 or 9, wherein the measuring device is an optical coherence tomography device.

11. The measuring device according to claim 10, wherein the optical coherence tomography device is used for examining a fundus.

FIG. 1

EP 2 833 168 A1

FIG. 2

## FIG. 3A

## FIG. 3B

## FIG. 3C

## FIG. 4A

## FIG. 4B

## FIG. 4C

FIG. 5A

FIG. 5B

FIG. 5C

FIG. 5D

FIG. 6

# FIG. 7

FIG. 8

TE POLARIZATION

TM POLARIZATION

AVERAGE

DIFFRACTION EFFICIENCY

100%
98%
96%
94%
92%
90%
88%

795
825
855
885
915

WAVELENGTH  nm

# FIG. 9

# FIG. 10

EP 2 833 168 A1

FIG. 11

EP 2 833 168 A1

Europäisches
Patentamt

European
Patent Office

Office européen
des brevets

## EUROPEAN SEARCH REPORT

Application Number

EP 14 17 8723

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HONGCHAO CAO ET AL: "Design and fabrication of a polarization-independent wideband transmission fused-silica grating", APPLIED OPTICS, OPTICAL SOCIETY OF AMERICA, WASHINGTON, DC; US, vol. 49, no. 21, 20 July 2010 (2010-07-20) , pages 4108-4112, XP001555736, ISSN: 0003-6935, DOI: 10.1364/AO.49.004108 [retrieved on 2010-07-19] | 1-9 | INV. G02B5/18 G02B27/42 G01B9/02 A61B5/00 |
| Y | * figures 1,3,5 * <br> * page 4109, left-hand column, paragraph 2 * <br> * page 4110, left-hand column, paragraph 1 * <br> * page 4109, right-hand column, paragraph 2 * <br> * figure 6 * <br> ----- | 10,11 | |
| Y | US 2010/245836 A1 (KULKARNI MANISH D [US] ET AL) 30 September 2010 (2010-09-30) <br> * paragraph [0027]; figure 1; table 2 * <br> * paragraph [0018] * <br> ----- | 10,11 | TECHNICAL FIELDS SEARCHED (IPC) <br><br> G02B <br> G01B <br> A61B |

The present search report has been drawn up for all claims

| Place of search | Date of completion of the search | Examiner |
|---|---|---|
| Munich | 14 November 2014 | Linke, Felix |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.**                    EP 14 17 8723

This annex lists the patent family members relating to the patent documents cited in the above-mentioned European search report.
The members are as contained in the European Patent Office EDP file on
The European Patent Office is in no way liable for these particulars which are merely given for the purpose of information.

14-11-2014

| Patent document cited in search report | Publication date | Patent family member(s) | Publication date |
|---|---|---|---|
| US 2010245836 A1 | 30-09-2010 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6747799 B

- JP 4749789 B